# EUROPEAN PATENT APPLICATION

(11) **EP 4 272 665 A1**
(43) Date of publication of application: **08.11.2023**
(21) Application number: 23171548.3
(22) Date of filing: 04.05.2023
(51) Int. Cl.: A61B 18/02, A61B 18/00

(54) **CRYOABLATION PROBE ASSEMBLY HAVING FLUID SHEATH**

(30) Priority: 04.05.2022 US 202263338180 P; 27.04.2023 US 202318308476
(71) Applicant: Medtronic, Inc., Minneapolis, Minnesota 55432 (US)
(72) Inventor: Skarda, James R., Brooklyn Park (US)
(74) Representative: Zimmermann & Partner Patentanwälte mbB

(57) **Abstract**

The disclosure relates to cryoablation probe assemblies including a cryoablation probe and sheath configured to accelerate thawing of the probe from frozen, treated tissue. The sheath can include a plurality of channels configured to direct saline or other biocompatible fluid to the treated tissue. The sheath is configured to slide and advance distally over the cryoablation probe to continually thaw the tissue until the cryoablation probe is freed. Methods of treating atrial fibrillation with a cryoablation probe assembly are also disclosed.

## Description

### FIELD

The present technology is generally related to cryoablation probe assemblies and methods of conducting cryoablation procedures that can be used to treat, for example, heart arrhythmias. The present technology is more specifically related to cryoablation probe assemblies that, for example, use heated fluid (e.g., saline) for thawing and detachment of a cryoablation probe from treated epicardial tissue.

### BACKGROUND

Atrial fibrillation is a type of heart arrhythmia or irregular heartbeat. In some circumstances, atrial fibrillation causes a decrease in the efficiency of the heart. In some circumstances, atrial fibrillation poses no immediate threat to the health of the individual suffering from the condition, but may, over time, result in conditions adverse to the health of the patient, including heart failure and stroke.

A variety of cardiac ablation devices and methods are currently available to treat atrial fibrillation and other arrhythmias. With some systems, endocardial tissue is contacted and ablated, for example via a cryoablation instrument via a standard sternotomy. Once the cryoablation instrument is positioned, cardiac tissue is frozen through cryogenic mechanism(s). Freezing destroys the treated tissue and helps to restore normal electrical conduction in the heart and to eliminate or reduce the arrhythmia.

### SUMMARY

The techniques described and otherwise disclosed generally relate to devices and methods for cryoablation procedures. Such devices and methods allow for a speedy detachment of a cryoablation probe from treated epicardial tissue and/or endocardial tissue by warming the tissue, for example, with fluid. Because several cryo-based lesions are typically completed per procedure, minimizing the overall cycle time is believed to provide significant efficiencies in the operating theater and reduces patient risk by reducing the overall procedure time.

One example provides a cryoablation probe assembly including a handle and a cryoablation probe supported by the handle. The assembly further includes a sheath coaxially positioned around the cryoablation probe. The sheath has a body defining a distal face, a central lumen and a plurality of channels extending from the distal face and through the body of the sheath. In addition, the assembly includes a fluid port in communication with the plurality of channels of the sheath. In some examples, a fluid source is connected to the fluid port. Optionally, the fluid source is in fluid communication with the central lumen. In some examples, the sheath is configured to slide with respect to the cryoablation probe.

Another example provides methods of conducting a cardiac cryoablation procedure. Such methods can include providing a cryoablation probe assembly including a handle and a cryoablation probe supported by the handle. The cryoablation probe including a bellows section including a first end and a second end. The assembly also includes a sheath coaxially positioned around the cryoablation probe. The sheath including a body defining a distal face, a central lumen and a plurality of channels extending from the distal face and through the body of the sheath. The assembly further includes a fluid port in communication with the plurality of channels of the sheath. The method includes positioning the bellows section of the cryoablation probe on cardiac tissue at a first location and freezing cardiac tissue at the first location to create a first transmural lesion. Additionally, the method includes directing fluid through the plurality of channels and through the distal face to the first end of the bellows section to release the first end of the bellows section from the cardiac tissue. The method further includes distally advancing the sheath and directing fluid through one or more of the plurality of channels or a central lumen of the sheath until the second end of the bellows is released from the cardiac tissue. The steps of forming lesions and freeing the cryoablation probe from frozen tissue can be repeated, as desired.

Further disclosed herein are cryoablation probe assemblies including a cryoablation probe and sheath configured to accelerate thawing of the probe from frozen, treated tissue, wherein the sheath can include a plurality of channels configured to direct saline or other biocompatible fluid to the treated tissue, wherein the sheath is configured to slide and advance distally over the cryoablation probe to continually thaw the tissue until the cryoablation probe is freed.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of a cryoablation probe assembly, according to some examples.
FIG. 2 is a partial, perspective view of a sheath of the cryoablation probe assembly of FIG. 1, according to some examples.
FIGS. 3A-3C depict one non-limiting example of a method of ablating cardiac tissue with the cryoablation probe assembly of FIG. 1.
FIG. 4A is a perspective view of a cryoablation probe assembly including an electrical heating element, according to some examples.
FIG. 4B is a partial, perspective view of a sheath of the cryoablation probe assembly including the electrical heating element of FIG. 4A, according to some examples.
FIG. 5 is a perspective view of a cryoablation probe assembly including an irrigated sheath, according to some examples.
FIG. 6 is a partial, perspective view of a sheath of the cryoablation probe assembly of FIG. 4A, according to some examples.

### DETAILED DESCRIPTION

Specific embodiments of the present disclosure are now described with reference to the figures, wherein like reference numbers indicate identical or functionally similar elements. The terms "distal" and "proximal" are used in the following description with respect to a position or direction relative to the treating clinician. "Distal" or "distally" are a position distant from or in a direction away from the clinician. "Proximal" and "proximally" are a position near or in a direction toward the clinician.

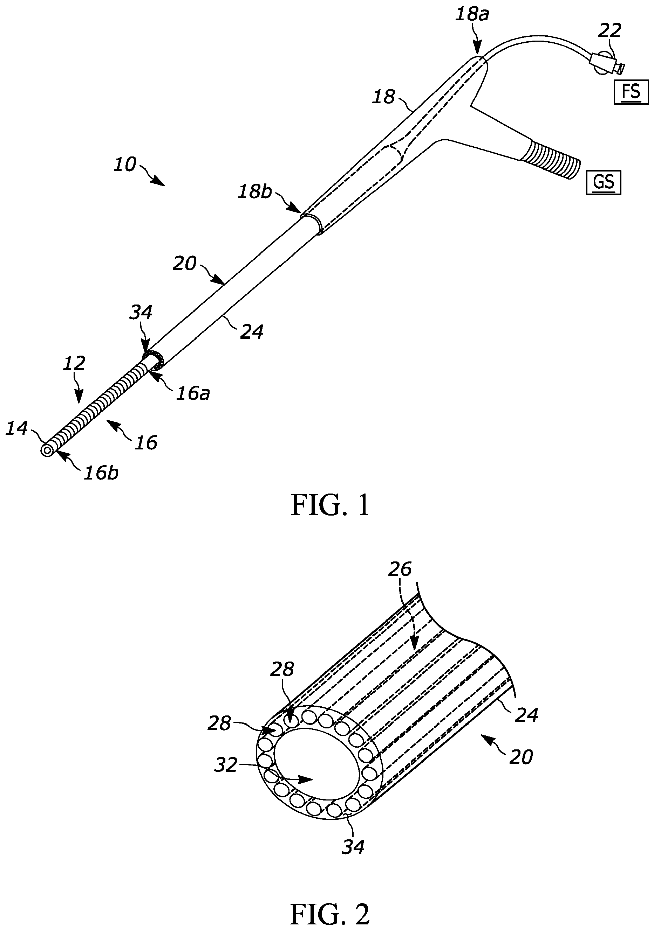

One illustrative example of a cryoablation probe assembly 10 of the disclosure is shown in FIGS. 1-2. In this example, the cryoablation probe assembly 10 includes a cryoablation probe 12, which can be any of those known in the art. One non-limiting cryoablation probe 12 includes a cryoablation probe, for example, model number 60SF2 available from Medtronic, Inc. of Minneapolis, Minnesota. Generally, the cryoablation probe 12 includes an elongated body 14 and a bellows section 16 having a first, proximal end 16a and a second, distal end 16b. Some portions of the body 14 can optionally be thermally insulating, however, the bellows section 16 is to be cooled when connected to a source of argon, nitrous or other cryo-thermal gas (schematically shown as a gas source, "GS"), for example, to freeze tissue during ablative procedures. Any other method of cooling the bellows section 16 is considered within the scope of the present disclosure. The assembly 10 further includes a handle 18 having a first end 18a and a second end 18b. The handle 18 supports the cryoablation probe 12 and can optionally serve as a connector to deliver argon gas or the like, which can be provided by a separate console or a wheeled cart supporting the argon tank and related electronics (not shown). It is envisioned that the cryoablation probe 12 is used once per patient and discarded as a biohazard while the console stays out of the sterile field and is used for many years. Once the cryoablation probe 12 is positioned, cardiac tissue is frozen through cryogenic mechanism(s) (e.g., isenthalpic free expansion of gas / Joule-Thomson effect or evaporation). The bellows section 16 of the cryoablation probe 12 must subsequently thaw before it can be removed from the treated tissue to either begin the next lesion or end the procedure and withdraw the assembly 10 from the patient. Any delay in thawing time inherently increases procedure time and any associated risks with extended procedure time.

Therefore, the assembly 10 additionally includes a sheath 20 configured to accelerate thawing and detachment of the cryoablation probe 12 from the tissue. The sheath 20 has a fluid port 22 and is coaxially positioned around the body 14 and covers a portion of a length of the body 14 between the bellows section 16 and the handle 18. In some examples, the sheath 20 can slide with respect to both the cryoablation probe 12 and the handle 18. In the illustrated example, the sheath 20 passes through the ends 18a, 18b of the handle 18 to slide and position a distal face 34 of the sheath 20 at various positions along a length of the body 14 of the cryoablation probe 12. The sheath 20 includes a body 24 and, optionally, a plurality of channels 26 extending through the body 24, each terminating at an opening 28 on the distal face 34. The sheath 20 further includes a central lumen 32 positioned within and defined by the body 14 for receiving the cryoablation probe 12. The central lumen 32 terminating at the distal face 34. Saline or the like can be directed from a fluid source (schematically shown as "FS"), though the fluid port 22, along the channels 26 and out of the openings 28 to dispense the saline (e.g., 0.9% normal saline) or other sterile biocompatible fluid (e.g., sterile water, sterile air, etc.) at the distal face 34, adjacent the bellows section 16. In this way, cardiac tissue adjacent the distal face 34 can be quickly thawed after being ablated so that the cryoablation probe 12 can be readily removed from the tissue for conducting an additional ablation or completion of the procedure. Because the sheath 20 can slide along the body 14 of the cryoablation probe 12, fluid can be continually dispersed to directionally thaw the tissue first from the first end 16a of the bellows section 16 and then to the distal end 16b of the bellows section 16 as fluid is dispersed and tissue thaws. In another example, the fluid port 22 can alternatively or additionally be fluidly connected to the central lumen 32 so that fluid can be directed though central lumen 32 to exit at the distal face 34 to directionally thaw tissue in a similar manner. In other words, as a proximal area of the tissue thaws, the sheath 20 at the distal face 34 can be distally advanced over the recently thawed tissue to further the thawing process. If not saline, any other biocompatible fluid can be used with the devices and methods of the disclosure.

The channels 26 can take many configurations to achieve the goal of dispensing thawing fluid around the circumference of the cryoablation probe 12. Generally, the sheath 20 includes at least four channels 26. In some examples, the plurality of channels 26 collectively span 360 degrees of the distal face 34. Optionally, the opening 28 of each of the plurality of channels 26 at the distal face 34 are equally spaced.

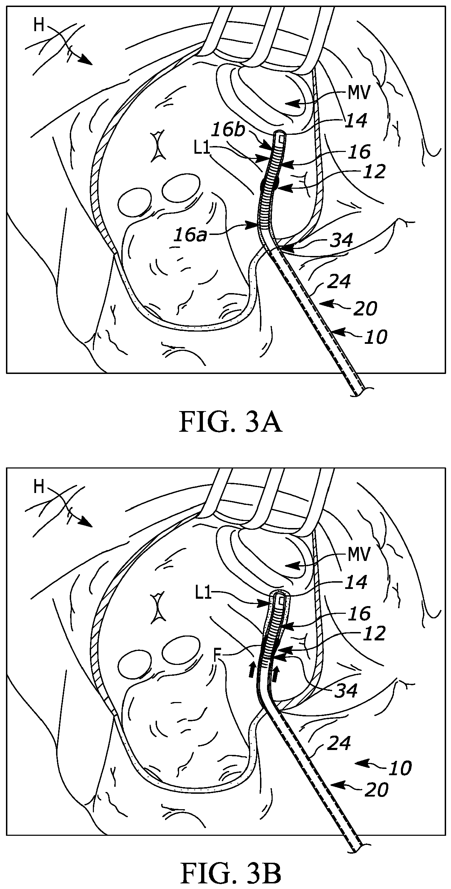

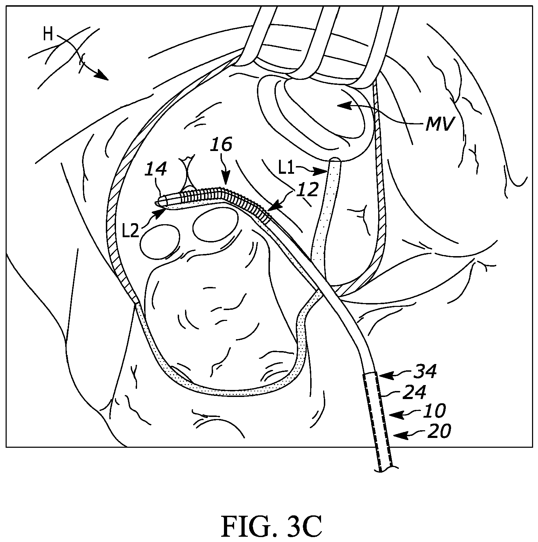

Referring in addition to FIGS. 3A-3C, yet other aspects in accordance with principles of the present disclosure relate to methods for ablating epicardial tissue of a patient to treat cardiac arrhythmia. These figures illustrate a heart H defined generally as being made of epicardial/cardiac tissue and having valves, including a visible mitral valve MV. In various methods, a cryoablation probe assembly 10 of any of the types disclosed herein is provided. The bellows section 16 of the cryoablation probe 12 is directed through an incision in a patient's chest (e.g., via a sternotomy), which may or may not be made for the delivery of a prosthetic heart valve (e.g., a prosthetic mitral valve at the native mitral valve MV). In such examples, the prosthetic heart valve may be delivered and deployed prior to arrhythmia treatment with the assembly 10. Upon delivery of the cryoablation probe assembly 10 to position the bellows section 16 at a first location L1 for forming a transmural lesion, cardiac tissue is frozen with the cryoablation probe 12. Once the transmural lesion is formed at the first location L1, fluid F, such as room temperature saline, is directed through the plurality of channels 26 and through the openings 28 in the distal face 34 to the first end 16a of the bellows section 16 to warm the tissue at the first end 16a and release the first end 16a from the tissue. As tissue continues to thaw, the sheath 20 can be further distally advanced to continually warm additional treated tissue and further distally advance the sheath 20 until the entire bellows section 16 is freed from the tissue. If desired, the bellows section 16 can be repositioned at a second location L2 in which a second transmural lesion can be formed. Once again, the bellows section 16 can be freed from adjacent tissue by thawing the tissue with fluid F directed from the sheath 20. The aforementioned steps can be repeated until the desired conductive block formed by multiple lesions has been completed.

In some instances, frost builds up, for example, on the bellows section 16. This frost build up is more likely to occur after consecutive ablative procedures. When frost build does occur, it becomes difficult for the sheath 20 to slide with respect to both the cryoablation probe 12 and the handle 18. When the build-up of frost occurs on the bellows section 16, the sheath 20 may not slide between the cryoablation probe 12 and the handle 18 (e.g., the sheath 20 is frozen in place). In some instances, the distal face 34 and the openings 28 also experience build-up of frost. When this occurs, the fluid F supplied from the channels 26 is blocked from exiting the openings 28.

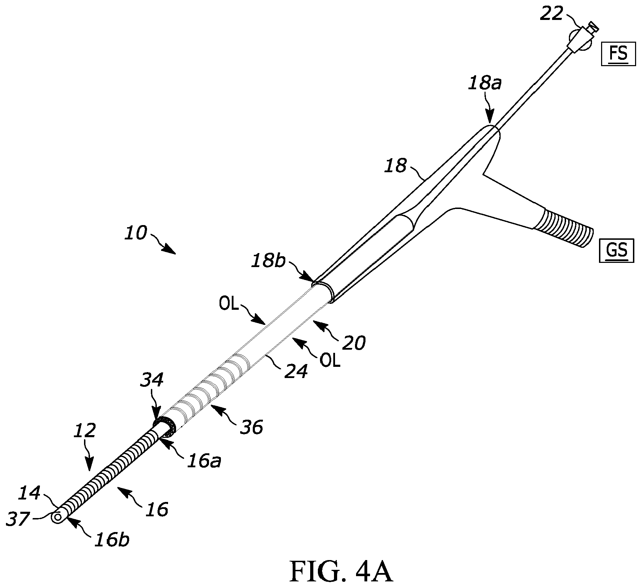

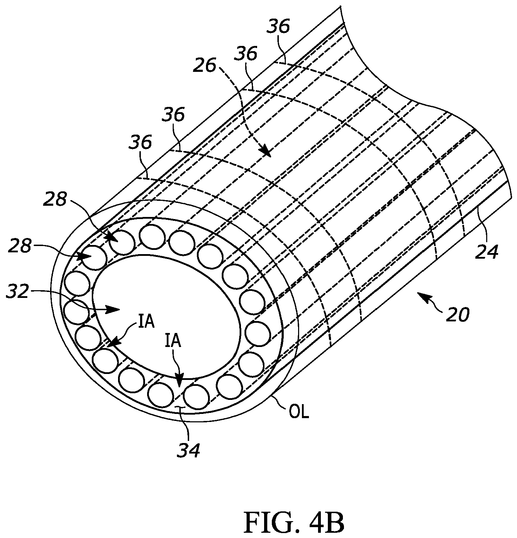

To help reduce problems caused by frost build-up, some examples include heating elements. One example is shown in FIGS. 4A-4B. In the example illustrated, the sheath 20 includes an electrical heating element 36. The electrical heating element 36 is configured to heat the sheath 20 and thaw the build-up of frost from the distal face 34 and the openings 28. In the example shown, the electrical heating element 36 is embedded within the sheath 20. The electrical heating element 36 is also positioned around the central lumen 32. In other examples, the electrical heating element 36 is embedded in, in some instances, or located under, in other instances, an outer surface or layer OL of the sheath 20. The outer surface or layer OL may be an outer wall of the sheath 20. In other examples, the sheath 20 is molded over (via a process commonly referred to as overmolding) the electrical heating element 36. By embedding the electrical heating element 36 in the outer surface or layer OL or overmolding the sheath 20 over the electrical heating element 36, heat from the electrical heating element 36 is directed toward an inner area IA of the sheath 20 (e.g., in a direction toward the central lumen 32). The outer surface or layer OL acts as an insulator and helps to maintain the outer layer OL at a lower temperature relative to the inner area IA. Maintaining the outer layer OL at a relatively lower temperature helps protect a user (e.g., a clinician) from touching a hot portion of the sheath 20 directly.

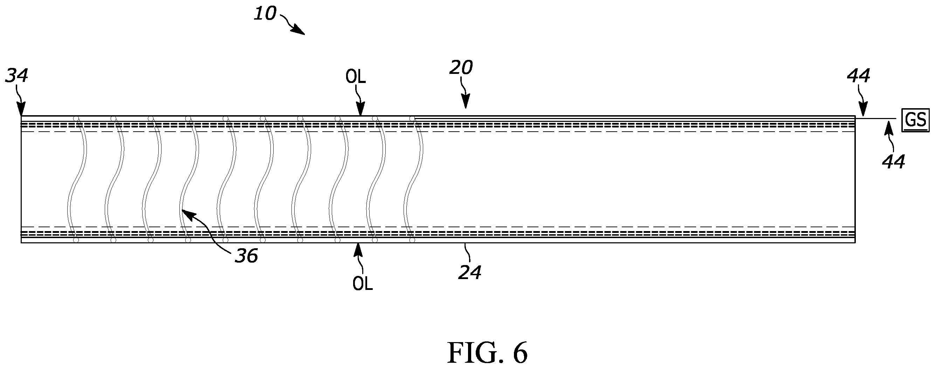

In the illustrated example, the electrical heating element 36 is a resistive heating element (e.g., a resistive heating coil that includes one or more loops). The electrical heating element 36 receives a current from a control unit (further described below with reference to FIG. 6). The current passes through the electrical heating element 36 and the electrical heating element 36 converts electrical energy of the current into heat (e.g., thermal energy) through a process of resistance (or Joule heating). The heat radiates through the sheath 20 to thaw the build-up of frost on the distal face 34 and the openings 28.

In other examples, the electrical heating element 36 is an inductive heating element (e.g., an inductive heating coil that includes one or more loops). An external electro-magnetic field generator creates an external electro-magnetic field . The external electromagnetic field induces currents inside the electrical heating element 36, for example, eddy currents. The eddy currents flow through the electrical heating element 36 and the electrical heating element 36 converts electrical energy of the currents to heat via Joule heating. The heat radiates through the sheath 20 to thaw the build-up of frost on the distal face 34 and the openings 28.

As an alternative to resistive and inductive heating elements in the sheath 20, some examples use external sources to provide heat to thaw tissue. In some instances, heating energy is applied using radio frequency heating, microwave heating, or ultrasonic heating from a suitable source, for example a radio frequency heating system, a microwave generator, or an ultrasonic heating system.

In some examples, the sheath 20 includes a thermally insulating material or layer located between the central lumen 32 and the outer layer OL. This insulating material or layer helps to maintain heat generated by the electrical heating element 36 in the sheath 20 and reduce heating of the outer layer OL. In some cases, the channels 26 act as the thermally insulating material. Typically, saline is directed through the channels 26. However, the insulating capability of the channels 26 is increased when sterile biocompatible fluid such as sterile air is directed from the fluid source FS through the channels 26.

In some examples, the cryoablation probe 12 includes a temperature sensor 37 at a distal tip of the body 14. The temperature sensor 37 is configured to sense a temperature of the body 14. When the cryoablation probe 12 is in use it contacts the patient's tissue and, therefore, the temperature sensor 37 provides an indication of the temperature of the tissue. The temperature sensor 37 is also configured to transmit a signal indicative of the temperature to the control unit.

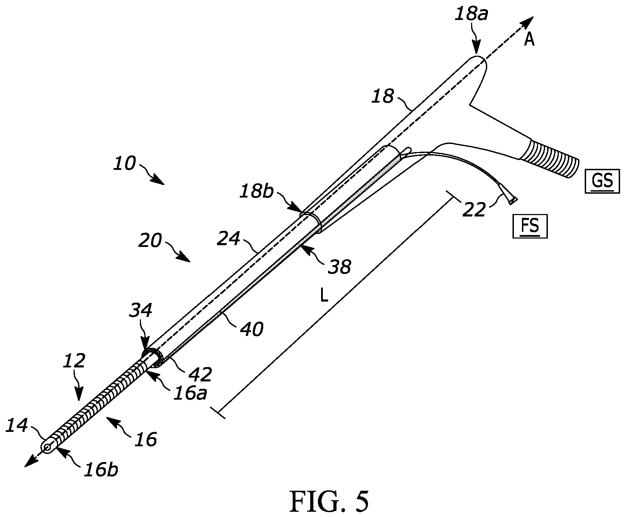

FIG. 5 illustrates an example of the sheath 20 that is an irrigated sheath. In the example shown, the fluid port 22 is connected to a fluid tube 38. The fluid tube 38 extends within the handle 18 parallel to a longitudinal axis A. In some examples, the fluid tube 38 extends within the handle 18 for a length L of the sheath 20. In some examples, the fluid tube 38 includes a plurality of holes 40. The plurality of holes 40 supply the fluid F to thaw the sheath 20.

In the example illustrated, the plurality of holes 40 are laser drilled into the fluid tube 38. Laser drilling is a preferred technique of creating the plurality of holes 40 because it is possible with laser drilling to create holes at a size small enough to evenly supply fluid F along the length L of the sheath 20. It is, however, possible to create the holes 40 using techniques other than laser drilling.

The fluid tube 38 also includes an end portion 42. The end portion 42 is positioned proximate the first end 16a of the bellows section 16. The end portion 42 supplies the fluid F to thaw the bellows section 16 and a portion of sheath 20 located near the first end 16a. Thawing the bellows section 16 aids in sliding the sheath 20 along the bellows section 16. The plurality of holes 40 and the end portion 42 direct the fluid F into the inner area IA of the sheath 20 and around the central lumen 32 to the first end 16a of the bellows section 16. The fluid F aids in releasing the first end 16a of the bellows section 16 from the cardiac tissue.

With reference to FIG. 6, the electrical heating element 36 is electrically connected to one or more wires and, in the example shown, two wires 44. In some examples, the wire 44 traverses the length L of the sheath 20. The wire 44 extends through the handle 18 to the control unit. For example, the wire 44 extends through the handle 18 and electrically connects to the control unit via the gas source GS (shown in FIGS. 4A and 5). In some examples, the control unit includes a user interface that receives a user input from the user. The control unit supplies the current to the electrical heating element 36 via the wire 44 based on a first user input. The first user input may be indicative of an on/off setting of the supply of current or a heating rate of the electrical heating element 36. In other examples, the control unit controls a flow rate of the fluid F based on a second user input. In other examples, the control unit receives the signal indicative of the temperature of the body 14 or the tissue. The control unit determines a thaw rate of the tissue based on the signal and controls the flow rate of the fluid F based on the thaw rate. In other instances, the flow rate of the fluid F is controlled manually. A user injects the fluid F into the sheath 20 via the fluid port 22, for example, via a syringe. The fluid F may be injected as a slow bolus injection or as a rapid bolus injection to the fluid port 22.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

Further disclosed herein is the subject-matter of the following clauses:
1. A cryoablation probe assembly comprising:
   a handle;
   a cryoablation probe supported by the handle;
   a sheath coaxially positioned around the cryoablation probe, the sheath having a body defining a distal face, a central lumen and a plurality of channels extending from the distal face and through the body of the sheath; and
   a fluid port in communication with the plurality of channels of the sheath.
2. The cryoablation probe assembly of clause 1, wherein the plurality of channels includes at least four channels.
3. The cryoablation probe assembly of clause 1 or of any of the preceding clauses, wherein the plurality of channels collectively span 360 degrees of the distal face.
4. The cryoablation probe assembly of clause 1 or of any of the preceding clauses, further comprising a fluid source connected to the fluid port.
5. The cryoablation probe assembly of clause 1 or of any of the preceding clauses, wherein an opening of each of the plurality of channels at the distal face are equally spaced.
6. The cryoablation probe assembly of clause 1 or of any of the preceding clauses, wherein the sheath is configured to slide with respect to the cryoablation probe.
7. The cryoablation probe assembly of clause 1 or of any of the preceding clauses, wherein the handle includes a first end and a second end and the sheath extends through both of the first and second ends.
8. The cryoablation probe assembly of clause 1 or of any of the preceding clauses, wherein the fluid port is in fluid communication with the central lumen.
9. A method of conducting a cardiac cryoablation procedure, the method comprising:
   providing a cryoablation probe assembly including:
      a handle,
      a cryoablation probe supported by the handle, the cryoablation probe including a bellows section including a first end and a second end,
      a sheath coaxially positioned around the cryoablation probe, the sheath including a body defining a distal face, a central lumen and a plurality of channels extending from the distal face and through the body of the sheath, and
      a fluid port in communication with the plurality of channels of the sheath,
   positioning the bellows section of the cryoablation probe on cardiac tissue at a first location;
   freezing cardiac tissue at the first location to create a first transmural lesion;
   directing fluid through the plurality of channels and through the distal face to the first end of the bellows section to release the first end of the bellows section from the cardiac tissue; and
   distally advancing the sheath and directing fluid through at least one of the plurality of channels or the central lumen until the second end of the bellows is released from the cardiac tissue.
10. The method of clause 9, further comprising repositioning the bellows section of the cryoablation probe to a second location; freezing the cardiac tissue to create a second transmural lesion; and directing fluid through the plurality of channels and through the distal face to release the cryoablation probe from the cardiac tissue.
11. The method of clause 9, further comprising creating additional transmural lesions to create a conduction block.
12. The method of clause 9, wherein the fluid is saline.
13. The method of clause 9, wherein the fluid is room temperature.
14. The method of clause 9, wherein the plurality of channels includes at least four channels.
15. The method of clause 9, wherein the plurality of channels collectively span 360 degrees of the distal face.
16. The method of clause 9, wherein an opening of each of the plurality of channels at the distal face are equally spaced.
17. The method of clause 9, wherein the handle includes a first end and a second end and the sheath extends through both of the first and second ends.
18. The method of clause 9, further comprising the step of directed fluid though the central lumen.
19. A cryoablation probe assembly comprising:
   a handle;
   a cryoablation probe supported by the handle;
   a sheath coaxially positioned around the cryoablation probe, the sheath having a body defining a distal face, a central lumen and a plurality of channels extending from the distal face and through the body of the sheath; wherein the sheath is configured to slide with respect to the cryoablation probe;
   a fluid port in communication with the plurality of channels of the sheath; and
   a fluid source connected to the fluid port and in fluid communication with the central lumen.
20. The cryoablation probe assembly of clause 19, wherein the plurality of channels includes at least four channels.
21. The cryoablation probe assembly of clause 1 or of any of the preceding clauses, further comprising an electrical heating element positioned within the sheath and around the central lumen, wherein the electrical heating element is configured to heat the sheath.
22. The method of clause 9, further comprising:
   directing fluid into an inner area of the sheath and around the central lumen to the first end of the bellows section to release the first end of the bellows section from the cardiac tissue.
23. The cryoablation probe assembly of clause 19, further comprising an electrical heating element positioned within the sheath and around the central lumen, wherein the electrical heating element is configured to heat the sheath.

## Claims

1. A cryoablation probe assembly comprising:
a handle;
a cryoablation probe supported by the handle;
a sheath coaxially positioned around the cryoablation probe, the sheath having a body defining a distal face, a central lumen and a plurality of channels extending from the distal face and through the body of the sheath; and
a fluid port in communication with the plurality of channels of the sheath.

2. The cryoablation probe assembly of claim 1, wherein the plurality of channels includes at least four channels.

3. The cryoablation probe assembly of any of the preceding claims, wherein the plurality of channels collectively span 360 degrees of the distal face.

4. The cryoablation probe assembly of any of the preceding claims, further comprising a fluid source connected to the fluid port.

5. The cryoablation probe assembly of any of the preceding claims, wherein an opening of each of the plurality of channels at the distal face are equally spaced.

6. The cryoablation probe assembly of any of the preceding claims, wherein the sheath is configured to slide with respect to the cryoablation probe.

7. The cryoablation probe assembly of any of the preceding claims, wherein the handle includes a first end and a second end and the sheath extends through both of the first and second ends.

8. The cryoablation probe assembly of any of the preceding claims, wherein the fluid port is in fluid communication with the central lumen.

9. The cryoablation probe assembly of any of the preceding claims, wherein the sheath is configured to slide with respect to the cryoablation probe; and the cryoablation probe assembly further comprises:
a fluid source connected to the fluid port and in fluid communication with the central lumen.

10. The cryoablation probe assembly of claim 9, wherein the plurality of channels includes at least four channels.

11. The cryoablation probe assembly of any of the preceding claims, further comprising an electrical heating element positioned within the sheath and around the central lumen, wherein the electrical heating element is configured to heat the sheath.

12. The cryoablation probe assembly of claim 9, further comprising an electrical heating element positioned within the sheath and around the central lumen, wherein the electrical heating element is configured to heat the sheath.
